# EUROPÄISCHE PATENTANMELDUNG

(11) **EP 1 195 439 A2**
(43) Veröffentlichungstag der Anmeldung: **10.04.2002**
(21) Anmeldenummer: 01120793.3
(22) Anmeldetag: 10.09.2001
(51) Int. Cl.: C12N 15/86, C12N 7/01, C12N 5/10, A61K 48/00, C07K 1/00

(54) **Retrovirale Vektoren mit Fremdsequenzinsertion zwischen retroviraler Primerbindungsstelle und retroviralem Spleissdonor**

(30) Priorität: 12.09.2000 DE 10045016
(71) Anmelder: Heinrich-Pette-Institut, 20251 Hamburg (DE)
(72) Erfinder: Baum, Christopher, Prof. Dr., 22589 Hamburg (DE); Schaumann, Daniel, 21465 Reinbek (DE)
(74) Vertreter: UEXKÜLL & STOLBERG

(57) **Zusammenfassung**

Gegenstand der Erfindung sind retrovirale Gentransfervektören, bei denen zwischen der retroviralen Primerbindungsstelle (PBS) und dem retroviralen Spleißdonor (SD) Fremdsequenzen eingeführt sind. Durch diese Modifikation wird die Effizienz der Genexpression verbessert, und die Vektoren zeichnen sich durch eine erhöhte Anwendungssicherheit aus.

## Beschreibung

Gegenstand der Erfindung sind retrovirale Gentransfervektoren, bei denen zwischen der retroviralen Primerbindungsstelle (PBS) und dem retroviralen Spleißdonor (SD) Fremdsequenzen eingeführt sind. Durch diese Modifikation wird die Effizienz der Genexpression verbessert, und die Vektoren zeichnen sich durch eine erhöhte Anwendungssicherheit aus.

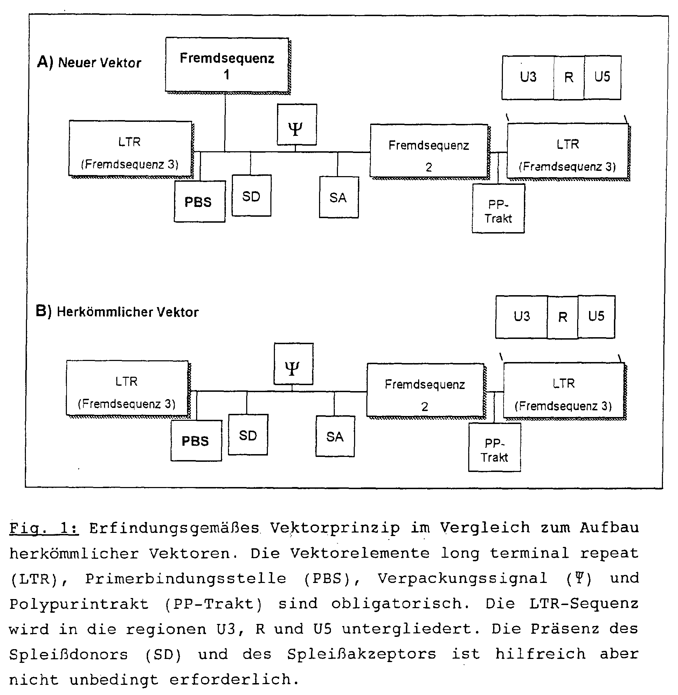

Herkömmliche retrovirale Vektoren haben den in Fig. 1 schematisch gezeigten Aufbau, wobei Fremdsequenzen entweder stromabwärts des Spleißakzeptorsignals (SA) stromabwärts des Spleißdonorsignals (SD) oder innerhalb der endständigen Sequenzwiederholungen (*long terminal repeat,* LTR) des retroviralen Vektors eingebaut sind.

Es besteht ein Bedarf an Vektoren, die sich durch eine verbesserte Genexpression und hohe biologische Sicherheit auszeichnen.

Diese Aufgabe wird erfindungsgemäß durch die retroviralen Vektoren der Patentansprüche 1 bis 8 gelöst.

Im Rahmen der vorliegenden Erfindung wurde überraschenderweise festgestellt, daß retrovirale Vektoren mit dem allgemeinen Aufbau 5'-[LTR] - [PBS] - [SD] - [ψ] - [SA] - [PP] - [LTR]-3', bei denen
- [LTR]: die endständige Sequenzwiederholung (*long terminal repeat,* LTR) des retroviralen Vektors ist,
- [PBS]: die retrovirale Primerbindungsstelle ist,
- [SD]: das retrovirale Spleißdonorsignal ist,
- [Ψ]: das retrovirale Verpackungssignal ist,
- [SA]: das retrovirale Spleißakzeptorsignal ist,
und
- [PP]: ein Polypurintrakt (PP-Trakt) ist,
gegenüber dem Stand der Technik wesentliche Vorteile aufweisen, wenn zwischen der retroviralen Primerbindungsstelle (PBS) und dem retroviralen Spleißdonor (SD) eine Fremdsequenz [FS1] insertiert ist, die aus der Gruppe bestehend aus Enhancer-PromotorSequenzen (z.B. vom Maus-spleen-focus-forming virus), Enhancer-Sequenzen (auch ohne Promotor), cDNA-Sequenzen (die für ein zu exprimierendes Protein kodieren), Sequenzen für Chromatin-Modifikation, wie z.B. Matrix-Anheftungsregionen (MAR, z.B. vom humanen Interferon-β-Gen), und/oder Sequenzen für RNA-Prozessierungssignale (z.B. posttranskriptionelles Regulatorelement des Woodchuck hepatitis virus) ausgewählt ist.

Als Fremdsequenz im Sinne der Erfindung ist jede DNA-Sequenz zu werten, die nicht auf natürlichem, evolutionärem Weg, sondern über gezielte molekulargenetische Methoden eingefügt wurde.

Wird zugleich der Enhancer/Promoter aus der U3-Region des LTR entfernt (Yu et al., 1986), zeichnet sich ein derart modifizierter Vektor durch erhöhte biologische Sicherheit aus, da die Vektorsequenz bei Infektion mit replikationskompetenten Retroviren nicht mobilisiert werden kann. Das Risiko der Aktivierung stromabwärts gelegener zellulärer Sequenzen ist gemindert. Gegenüber herkömmlichen Konstrukten zeichnet sich der erfindungsgemäße Vektor durch eine mindestens gleichwertige Effizienz der Genexpression aus.

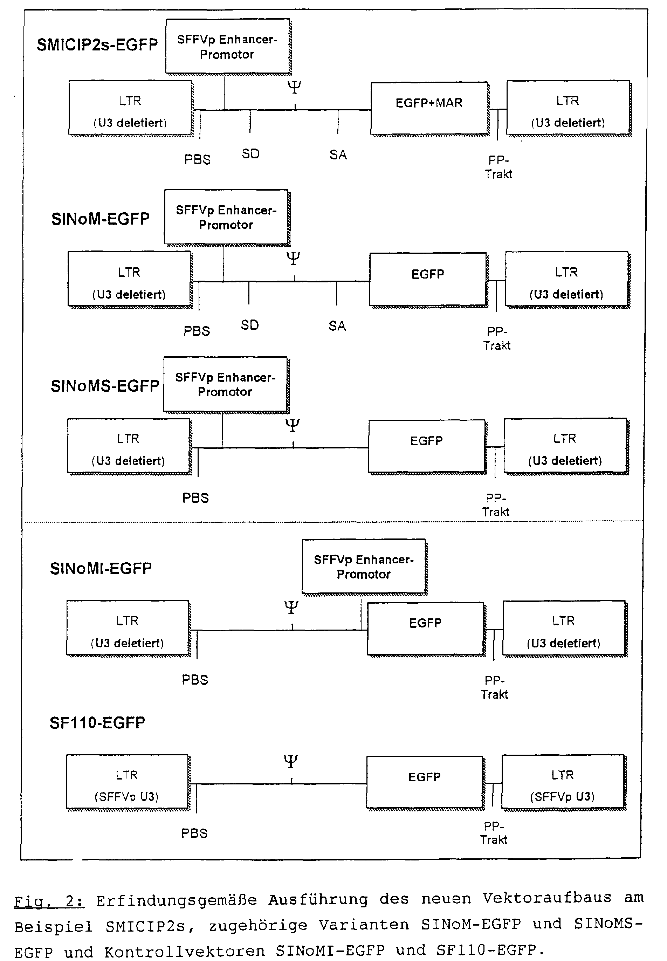

Die beschriebene Modifikation gegenüber Vektoren im Stand der Technik besteht darin, daß die erfindungsgemäßen Vektoren eine Fremdsequenz [FS1] enthalten, die stromaufwärts des retroviralen Spleißdonorsignals (SD) - das sich noch vor dem Verpackungssignal befindet (vgl. Fig. 1 und 2) - und stromabwärts der retroviralen Primerbindungsstelle (PBS) eingefügt ist. Stromabwärts des Spleißdonors oder Verpackungssignals kann, wie in herkömmlichen retroviralen Vektoren, eine zweite Fremdsequenz eingefügt werden (Fremdsequenz 2, FS2) (Hildinger et al., 1999; Deffaud und Darlix, 2000). Die Insertion von [FS2] erfolgt vorzugsweise zwischen [SA] und [PP], wobei die Präsenz des [SA] keine notwendige Voraussetzung für die erfindungsgemäßen Vektoren ist. Die endständige Sequenzwiederholung (*long terminal repeat,* LTR) des retroviralen Vektors kann ebenfalls derartige Fremdsequenzen (FS3) enthalten. Erfindungsgemäß kann der Vektor als Fremdsequenz nach obiger Definition entweder nur [FS1] oder aber [FS1] in Kombination mit [FS2] und/oder [FS3] enthalten.

Die Fremdsequenz [FS2] wird vorzugsweise aus der Gruppe bestehend aus cDNA-Sequenzen (z.B. kodierend für enhanced green fluorescent protein), Enhancer- und/oder Promotor-Sequenzen, Sequenzen für interne Ribosomeneintrittstellen (IRES), Sequenzen für Matrix-Anheftungsregionen (MAR, z.B. vom humanen Interferon-β-Gen) und/oder Sequenzen für RNA-Prozessierungssignale (z.B. posttranskriptionelles Regulatorelement des Woodchuck hepatitis virus) ausgewählt.

Die Fremdsequenz [FS3] wird vorzugsweise aus der Gruppe bestehend aus Klonierungsstellen zur Deletion des LTR-Enhancer-Promotors, Enhancer- und/oder Promotor-Sequenzen, cDNA-Sequenzen, Sequenzen für Matrix-Anheftungsregionen (MAR, z.B. vom humanen Interferon-β-Gen) und/oder Sequenzen für RNA-Prozessierungssignale (z.B. posttranskriptionelles Regulatorelement des Woodchuck hepatitis virus) ausgewählt.

Gemäß einer bevorzugten Ausführungsform der Erfindung enthält der Vektor folgende Fremdsequenzen:
- [FS1]: Fragment aus dem LTR des Maus-spleen-focus-forming-Retrovirus (SFFVp), das den retroviralen Enhancer-Promotor und die ersten 27 Basenpaare der 5'-untranslatierten Region des Retrovirus enthält (Baum et al., 1995).
- [FS2]: Kodierende Sequenzen (cDNA) des enhanced green fluorescent proteins (EGFP; Yang et al., 1996) mit 3' anschließendem, nicht-kodierenden Genfragment des humanen Interferon-β-Gens, das eine Matrix-Anheftungsregion (MAR) enthält (Schübeler et al., 1996).
- [FS3]: Künstliche Klonierungsstelle zur Deletion des LTR-Enhancer-Promotors (vgl. Yu et al., 1986).

Dieser Vektor, genannt SMICIP2s(eGFP), enthält 5' des Verpackungssignals den retroviralen Spleißdonor und 3' des Verpakkungssignals eine Spleißakzeptorsequenz (SA). Zu diesem Vektor (SMICIP2s(eGFP)) existieren zwei bevorzugte Varianten und zwei Kontrollvektoren:
- Variante 1: wie SMICIP2s(eGFP), jedoch fehlen die MAR-Sequenzen (Vektor SINoM-EGFP);
- Variante 2: wie Variante 1, jedoch fehlen zusätzlich die Spleißdonor- bzw. Spleißakzeptorsequenzen (Vektor SINoMS-EGFP);
- Kontrollvektor 1: der Enhancer-Promotor in der Fremdsequenz 1 wurde deletiert und stattdessen vor die EGFP-CDNA in die Fremdsequenz 2 eingefügt (Vektor SINoMI-EGFP) - repräsentiert herkömmliche Vektoren mit selbstinaktivierendem LTR (Yu et al., 1986);
- Kontrollvektor 2: repräsentiert den herkömmlichen Vektortypus mit intaktem LTR (Fig. 1B) ohne Fremdsequenz 1 (Vektor SF110-EGFP; Hildinger et al., 1999).

Diese Vektoren wurden als provirale Plasmide auf der Grundlage von pUC19 in E.coli kloniert. Die Plasmide enthalten in der 5'-LTR den Enhancer-Promotor (U3-Region) des MPSV-Retrovirus. Diese Sequenz liegt stromaufwärts der Cap-Stelle und findet sich daher nach Transfektion in eukaryontische Verpackungszellen im retroviralen Transkript nicht wieder. In retroviral transduzierten Zielzellen konstituiert sich das retrovirale Transgen entsprechend dem in Fig. 1 gezeigten Aufbau, wobei sich die LTR-Sequenzen bezüglich der U3-Region des Vektors aus dem 3'-LTR des Plasmids herleiten, die R- und U5-Sequenzen hingegen aus dem 5' LTR des Plasmids. Das provirale Plasmid des Vektors SMICIP2s(eGFP) heißt pSMICIP2s(eGFP); die Sequenz dieses Plasmids ist nachfolgend im Sequenzprotokoll wiedergegeben. Eine Probe dieses Plasmids wurde am 31. August 2000 bei der Deutschen Sammlung von Mikroorganismen und Zellkulturen (DSMZ), Mascheroder Weg 1b, 38124 Braunschweig unter der Nr. DSM 13711 hinterlegt.

Der Vektor SMICIP2s(eGFP) zeichnet sich dadurch aus, daß
1) ein starker retroviraler Enhancer/Promoter des SFFVp-Retrovirus (Baum et al., 1995) zwischen PBS und SD eingefügt wurde (=FS1),
2) als [FS2] stromabwärts des [SA] und stromaufwärts des [PP] die EGFP cDNA mit anschließender MAR-Sequenz des menschlichen Interferon-beta-Genes (Schübeler et al., 1996) verwendet wurde, und
3) als [FS3] eine Restriktionsstelle vorliegt, die den Enhancer/Promoter der U3-Region des LTRs ersetzt.

In dieser beispielhaften Kombination lassen sich infektiöse Titer und Expressionseigenschaften in den transduzierten Zielzellen erzielen, die herkömmlichen Vektoren ohne [FS1] mindestens vergleichbar sind. Dabei sind die sicherheitsrelevanten Merkmale des neuartigen Vektors den herkömmlichen Konstrukten überlegen. Erstmals ist es in der im Vektor SMICIP2s(eGFP) vorliegenden Sequenzkombination gezeigt worden, daß ein retroviraler Vektor einen intern zwischen den LTR-Sequenzen gelegenen Enhancer/Promoter mit nachgeschaltetem Intron in der 5'-untranslatierten Region enthalten kann, wobei die Insertion der Fremdsequenzen in der sense-Orientierung der retroviralen RNA erfolgt.

Gemäß einer besonderen Ausführungsform betrifft die Erfindung ferner einen Vektor, bei dem das retrovirale Spleißdonorsignal (SD) und/oder das retrovirale Spleißakzeptorsignal (SA) durch Mutation inaktiviert ist/sind.

Das zur erfindungsgemäßen Vektorkonstruktion verwendete Retrovirus ist aus der Gruppe bestehend aus Mausleukämieviren, Vogelretroviren (einschließlich spleen necrosis virus), Spumaviren (einschließlich humanes Foamyvirus), B-Typ-Retroviren (einschließlich Maus-Mammatumorvirus), D-Typ-Retroviren (einschließlich Mason-Pfizer-Affenvirus) oder Lentiviren (einschließlich human immunodeficiency virus I) ausgewählt. Diese Retroviren zeigen alle einen genetisch konservierten Aufbau bezüglich der Abfolge PBS gefolgt von SD und Verpackungssignal, und sie sind daher für die erfindungsgemäße Insertion der [FS1], mit oder nachgeschaltetem Intron, geeignet.

Gegenstand der vorliegenden Erfindung ist ferner ein infektiöses Viruspartikel, das den erfindungsgemäßen retroviralen Vektor enthält.

Zur Expression der von [FS1], [FS2] und/oder [FS3] kodierten Proteine kann der Vektor in eine Wirtszelle eingebracht werden, die bei Kultivierung unter geeigneten Bedingungen das gewünschte Protein exprimiert. Gegenstand der Erfindung sind somit ferner Wirtszellen, die mit dem erfindungsgemäßen Vektor bzw. mit den genannten infektiösen Viruspartikeln infiziert sind. Bei den Wirtszellen handelt es sich vorzugsweise um lymphatische, hämatopoetische oder mesenchymale Zellen.

Der retrovirale Vektor der vorliegenden Erfindung kann universell eingesetzt werden. Mögliche Anwendungsgebiete sind die Gentherapie, die Klonierung von Genen oder die Expression und/oder Überexpression von Proteinen oder RNA. Ferner eignet sich der Vektor besonders zur Transfektion lymphatischer, hämatopoetischer oder mesenchymaler Zellen.

Die vorliegende Erfindung betrifft ferner ein Verfahren zur Gewinnung von Proteinen, bei dem man eine vorgenannte Wirtszelle in einem geeigneten Medium unter Bedingungen kultiviert, die zur Expression der von den Fremdsequenzen [FS1], [FS2] und/oder [FS3] kodierten Proteinen notwendig sind, wobei man das so erzeugte Protein von den Zellen und dem Medium abtrennt.

Im Rahmen der Gentherapie kann der retrovirale Vektor ebenfalls eingesetzt bzw. zur Herstellung eines pharmazeutischen Präparates zur Gentherapie verwendet werden, wobei der Vektor in geeigneter Form vorliegt, um in die Zielzellen eingeführt zu werden.

Gegenstand der Erfindung ist ferner ein pharmazeutisches Präparat, das einen vorgenannten Vektor zusammen mit pharmazeutisch verträglichen Hilfs- und Trägerstoffen enthält. Dieses Präparat dient der Transfektion entsprechender Zielzellen *in vitro* und *in vivo.* Im Rahmen des gentherapeutischen Regimes können auch mit dem Vektor (*in vitro*) transfizierte lymphatische, hämatopoetische oder mesenchymale Zellen als pharmazeutisches Präparat angesehen und appliziert werden.

Die im Rahmen der vorliegenden Erfindung durchgeführte Experimente haben gezeigt, daß die Fremdsequenz 1 (FS1) unter Erhalt der genetischen Stabilität des retroviralen Vektors eingefügt werden kann. Durch die erfindungsgemäße Insertion der Fremdsequenz 1 ergibt sich eine Vielfalt neuartiger Gestaltungsmöglichkeiten retroviraler Vektoren. Im folgenden sind einige Beispiele aufgeführt, wie der erfindungsgemäße Einbau einer FS1 zwischen PBS und SD die Gestaltungsmöglichkeiten retroviraler Vektoren erweitert.
o Bei Erhalt des Enhancer/Promotors im LTR kann als FS1 eine cDNA eingefügt werden. Die FS2 kann dann eine zweite Expressionskassette enthalten. Dabei kann die Verknüpfung der Expression mit FS2 entweder durch einen internen Promotor oder eine interne Ribosomeneintrittstelle vor der cDNA in FS2 geschehen kann. Damit wird die Möglichkeit der gleichzeitigen Expression mehrerer cDNAs von einem Vektor verbessert.
o Es ist auch möglich, in der FS1 eine reine Enhancersequenz einzusetzen, die entweder auf einen Promotor im LTR (FS3) oder auf einen internen Promotor in FS2 expressionsfördernd wirkt.
o Die Einfügung einer Matrix-Anheftungsregion in der FS1 kann sich förderlich auf die Expression eines Vektors auswirken, der einen internen Promotor mit nachfolgender cDNA in der FS2 oder eine Kombination von Promotor und cDNA als FS3 im LTR trägt.
o Liegt ein Vektor vor, der einen Promotor mitsamt nachfolgender cDNA als FS3 im LTR trägt, kann der Einbau eines RNA-Prozessierungssignals (z.B. posttranskriptionelles Regulatorelement des Woodchuck hepatitis virus) in der FS2 expressionsfördernd wirken, ähnlich wie für konventionelle Vektoren gezeigt (Zufferey et al., 1999).

Die vorliegende Erfindung wird nachfolgend anhand von Beispielen, Figuren und einem Sequenzprotokoll erläutert.

### BEISPIELE

### Beispiel 1:

### Herstellung eines erfindungsgemäßen Vektors:

### 1. Ausgangskonstrukt

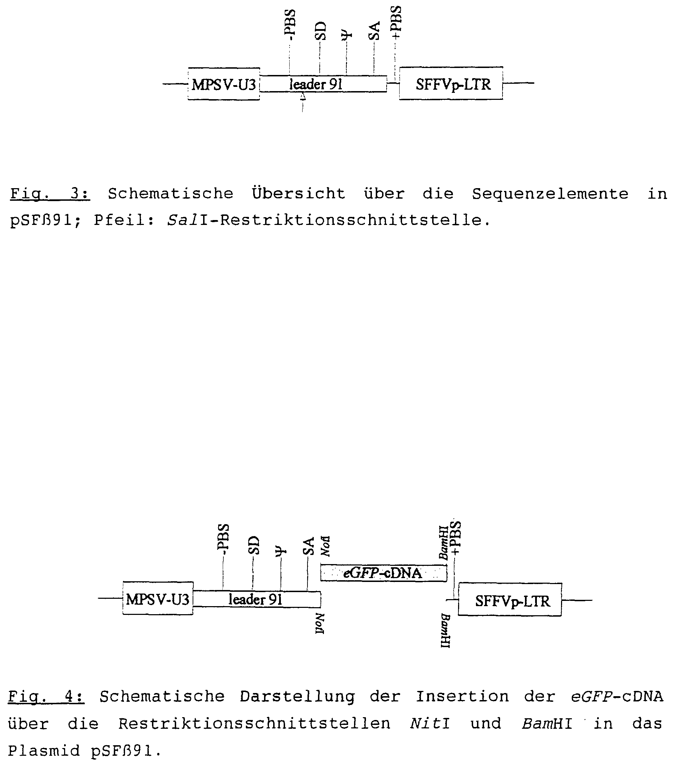

Die molekularbiologische Konstruktion des retroviralen Vektors vollzog sich auf der Ebene bakterieller Plasmide, die die proviralen Sequenzen enthalten. Das hier verwendete Ausgangskonstrukt leitet sich vom Plasmid pSFβ91 ab (Hildinger et al., 1999; Fig. 3), das den 3'-LTR aus dem spleen focus-forming virus (SFFVp), die 5'-U3 vom MPSV und die 5'-untranslatierte Region (leader) des murine embryonic stem cell virus (MESV) enthält. pSFβ 91 enthält eine modifizierte leader-Region, die sich in der Anwesenheit zweier Spleißsignale auszeichnet, namentlich den Spleißdonor (SD) und den Spleißakzeptor (SA; Fig. 3).

Am 3'-Ende der leader-Region befinden sich Erkennungsstellen für die Restriktions-Endonucleasen *Not*I, *Eco*RI, *Bam*HI und *Hind*III. Gegenüber diesen Plasmiden von Hildinger et al. (1999) besitzen die verwendeten Konstrukte zusätzlich eine *Sal*I-Restriktionsstelle downstream von der -PBS an der Basenposition 1030 (Fig. 3, Pfeil).

Im folgenden wurden in diese Plasmide die cDNA des enhanced green flourescent protein (eGFP) und ein 800 bp-großes Fragment der Kernmatrix-Anheftungsregion aus dem humanen β-Interferon Gen-Locus eingeführt. Desweiteren wurden die *cis*-aktiven Elemente der U3-Region im 3'-LTR deletiert und die eGFP-cDNA unter die transkriptionelle Kontrolle der U3-Region des SFFVp gestellt, die in der leader-Region 5'-wärts vom SD inseriert wurde.

### 2. Einsetzen der eGFP-cDNA

Das Einsetzen der *eGFP*-cDNA aus dem Plasmid pMP110(eGFP) (Schambach et al., 2000) in pSFβ91 erfolgte über die Restriktionsstellen von *Not*I und *Bam*HI am 5'-Ende der leader-Region (Fig. 4). Es entstand das Plasmid pSFβ91(eGFP).

### 3. Deletion der cis-aktiven Elemente der U3-Region im 3'-LTR

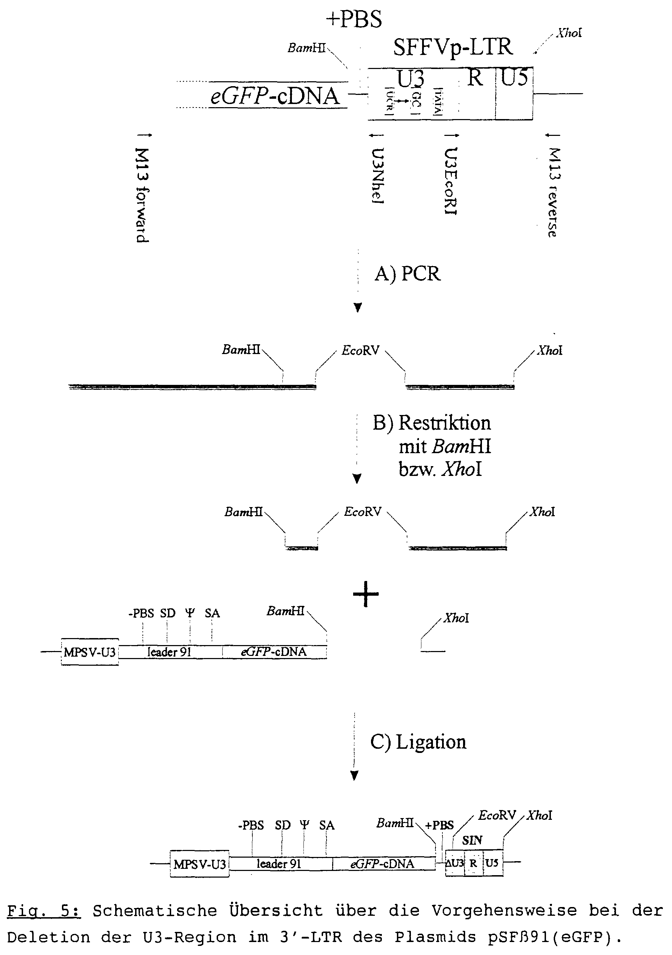

Für die Deletion der *cis*-aktiven Elemente in der U3-Region des 3'-LTR wurden über PCR zwei Fragmente aus dem Plasmid pSFβ91(eGFP) amplifiziert (Fig. 5A). Das eine Fragment stellt den Bereich zwischen dem 5'-Ende des 5'-LTR und dem 5'-Ende der 3'-LTRs dar. Als Primer für die Vorwärtsstrang-Synthese diente das Oligonucleotid M13forward, für die Synthese des Rückwärtsstranges das Oligonucleotid U3NheI. U3NheI besitzt am 5'-Ende eine Restriktionsschnittstelle für *Nhe*I, so daß das Amplifikat ebenfalls diese Schnittstelle am 3'-Ende aufwies. Nach Restriktion des Amplifikats mit dem Enzym *Bam*HI blieb ein 80 bp-großes Fragment übrig, daß das 5'-Ende der U3-Region und den PP umfaßte (Fig. 5B, +PBS).

Für die Amplifikation des anderen Fragments dienten die Primer M13reverse (Rückwärtsstrang-Synthese) und U3EcoRI (Vorwärtsstrang-Synthese). Sie hybridisieren sequenzspezifisch am 3'-Ende der U3-Region (U3EcoRI) und downstream vom 3'-LTR (M13reverse; Fig. 5A). Durch U3EcoRI, der eine *Eco*RI-Restriktionsschnittstelle am 5'-Ende besitzt, wurde am 5'-Ende des Amplifikats eine *Eco*RI-Schnittstelle eingefügt. Durch Restriktion des Amplifikats mit *Xho*I entstand ein 170 bp-großes Fragment, das neben dem 3'-Ende der U3-Region die R- und die U5-Region enthielt (Fig. 5B).

Die beiden geschnittenen Amplifikate wurden nacheinander über die Restriktionsschnittstellen *Bam*HI und *Eco*RV bzw. *Eco*RV und *Xho*I in den Klonierungsvektor pBluescript SK II eingesetzt, um dann mit Hilfe der Restriktionsenzyme *Bam*HI und *Xho*I als ein Fragment ausgeschnitten zu werden. Die Oligonucleotide U3NheI und U3EcoRI enden am 5'-Ende jeweils mit dem Basentriplett ATC, so daß bei der bei der blunt end-Verknüpfung beider geschnittenen Amplifikate eine *Eco*RV-Schnittstelle entstand (Fig. 5; Sequenz: GATATC).

### Durchführung der PCR:

- Ansatz:: 10 ng DNA
1 µl Pfu Turbo™-Polymerase (1 U/µl) (Hersteller: Stratagene, La Jolla, US)
100 pmol Primer 1
100 pmol Primer 2
5 µl dNTP-Mix (je 10 mM) (Hersteller: Qiagen, Hilden, DE)
5 µl 10 x Cloned-Pfu-Puffer (100 mM KCl; 100 mM (NH₄)₂SO₄; 200 mM Tris-HCl, pH 8.5; 20 mM MgSO₄; 1% w/v Triton X-100; 1 mg/ml BSA) (Stratagene) ad. 50 µl Aqua dest.

Es wurde am Thermo-Cycler (Biometra, Göttingen) folgendes Programm gewählt:

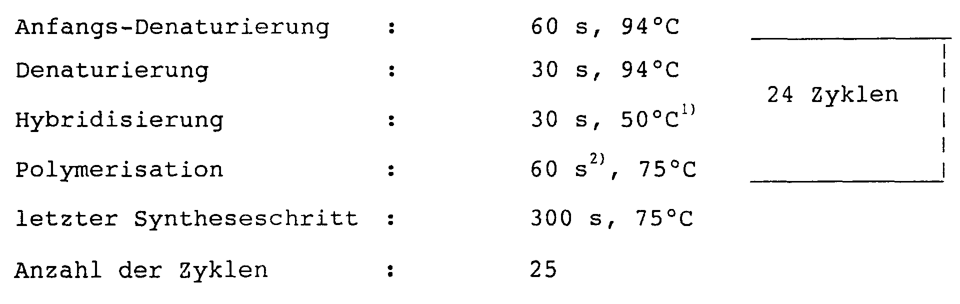

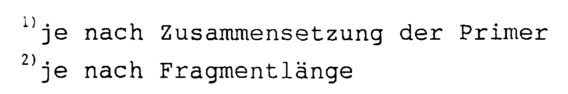

Als Negativ-Kontrollen für die PCR diente ein Ansatz mit nur je einem der beiden Primer und ein Ansatz ohne Primer. Für die Positiv-Kontrolle wurde ein definiertes Plasmid mit speziellen Primern eingesetzt, das bei erfolgreicher Durchführung der PCR ein Amplifikationsprodukt bekannter Größe liefert.

Die für die PCR verwendeten Oligonukleotid-Primer wurden von der Firma Gibco BRL hergestellt.

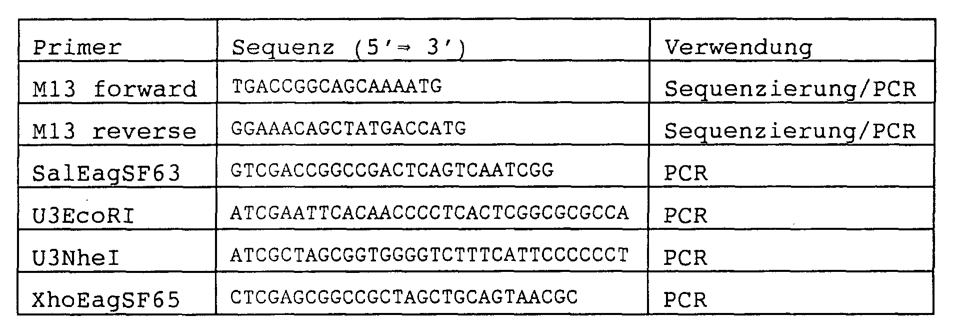

Aus dem Plasmid pSFβ91(eGFP) wurde mit *Bam*HI und *Xho*I der Bereich downstream von der *eGFP*-cDNA ausgeschnitten und durch die miteinander verknüpften PCR-Fragmente ersetzt (Fig. 5C). Die entstandenen Plasmide pSIN91(eGFP) bzw. pSIN110(eGFP) enthalten in der U3-Region des 3'-LTR keine Enhancer- oder Promotor-Elemente.

### 4. Insertion des Promotors

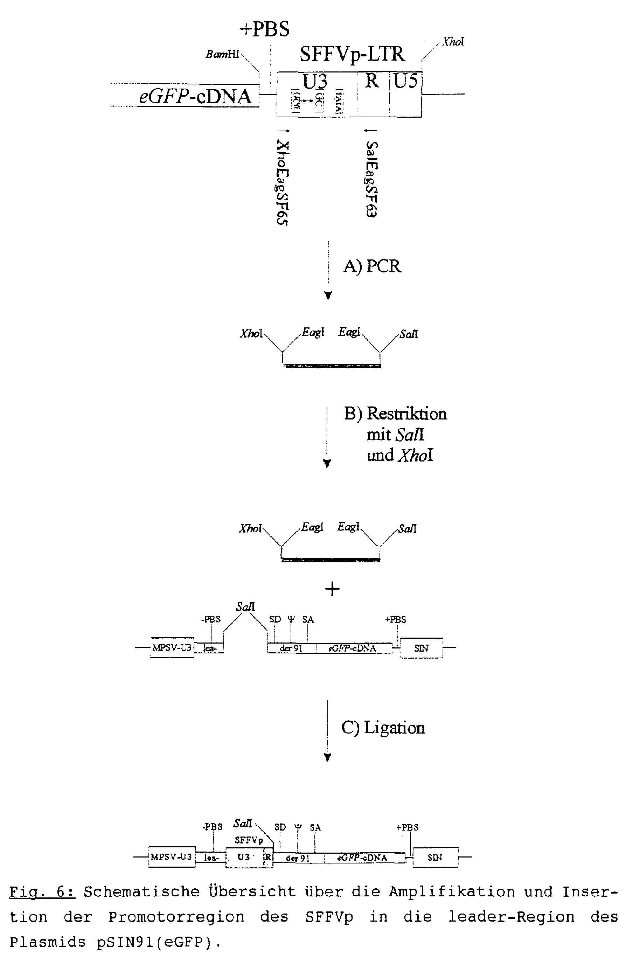

Nachdem im 3'-LTR die *cis*-aktiven Sequenzen deletiert worden sind, erfolgte die Insertion des Promotors in der leader-Region vor die SD-Stelle über die *Sal*I-Restriktionsschnittstelle (Fig. 6). Das Promotorfragment wurde zunächst über PCR mit den sequenzspezifischen Primern XhoEagSF65 und SalEagSF63 amplifiziert, die am 5'-Ende des 3'-SFFVp-LTRs (XhoEagSF65) bzw. in dem Bereich ca. 30 bp downstream vom 5'-Ende der R-Region (SalEagSF63) hybridisieren (Fig. 6A). Das Amplifikat umfaßte deshalb neben der gesamten U3-Region auch die ersten 27 Nucleotide der R-Region, die das Cap-Signal enthalten und daher für eine optimale Translation der mRNA unverzichtbar sind (Cupelli und Lenz, 1991).

Die für die PCR verwendeten Oligonukleotide weisen an ihrem 5'-Ende Erkennungsstellen für die Restriktions-Endonukleasen *Xho*I und *Eag*I (XhoEagSF65) bzw. *Sal*I und *Eag*I (SalEagSF63) auf, so daß das Amplifikat eben diese Schnittstellen am 5'- bzw. 3'-Ende trug. Um das PCR-Produkt über die *Sal*I-Restriktionsstelle in den Vektor zu inserieren, wurde es zuvor mit *Xho*I und *Sal*I geschnitten (Fig. 6B). Aufgrund der Komplementarität der Basenüberhänge von *Xho*I und *Sal*I nach einer Restriktion ist dieses Vorgehen möglich. Durch das Primerdesign wurde erreicht, daß nach der Ligation nur auf jeweils einer Seite eine *Sal*I-Restriktionsstelle wiederhergestellt wurde (Fig. 6C) und bei zukünftigen Klonierungen für die Insertion weiterer Fragmente wiederverwendet werden kann, ohne dabei den Promotor wieder auszuschneiden.

### 5. Insertion der Kernmatrix-Anheftungsregion (MAR)

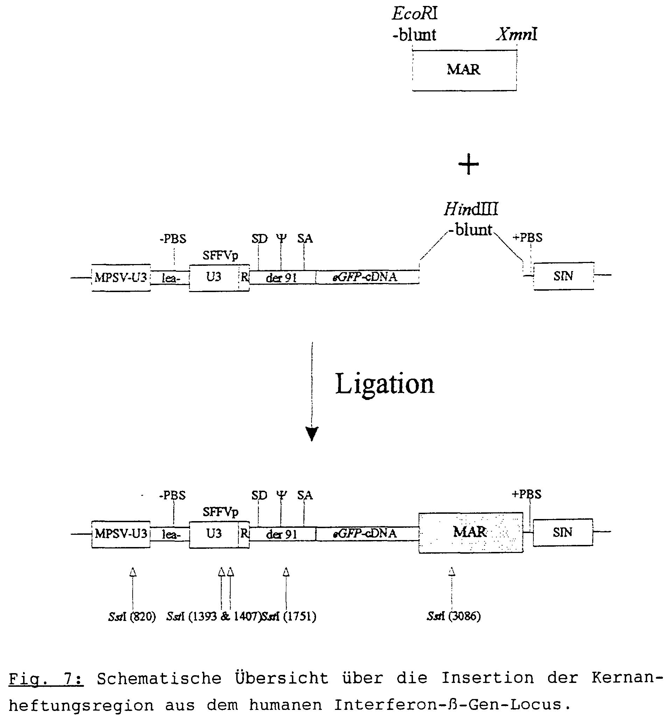

Ein ca. 800 bp-großes Fragment der Kernmatrixanheftungs-Region aus dem humanen Interferon-β Gen-Locus wurde aus dem Plasmid pTZE20 (Mielke et al., 1990) mit Hilfe der Restriktions-Endonucleasen *Xmn*I und *Eco*RI ausgeschnitten. Mittels der Klenow-Polymerase wurde der vier Nucleotid-große Basenüberhang der *EcoRI*-Schnittstelle zu einem glatten Ende aufgefüllt, so daß das Restriktionsfragment an beiden Flanken blunt ends besaß. Die MAR wurde direkt am 3'-Ende der eGFP-cDNA über die *Hin*dIII-Schnittstelle eingesetzt, deren Basenüberhänge nach dem Schnitt ebenfalls mit der Klenow-Fragment der DNA-Polymerase I von *E. coli* zu glatten Enden aufgefüllt wurden (Fig. 7). Die MAR wurde dabei in sense-Orientierung zum Promotor in den Vektor inseriert, was durch eine Restriktionsspaltung mit der Endonuklease SstI verifiziert wurde, die die MAR in zwei unterschiedlich große Fragmente schneidet. Sie liegt damit in derselben Orientierung zum Promotor wie im humanen Interferon-β Gen-Locus (Mielke et al., 1990).

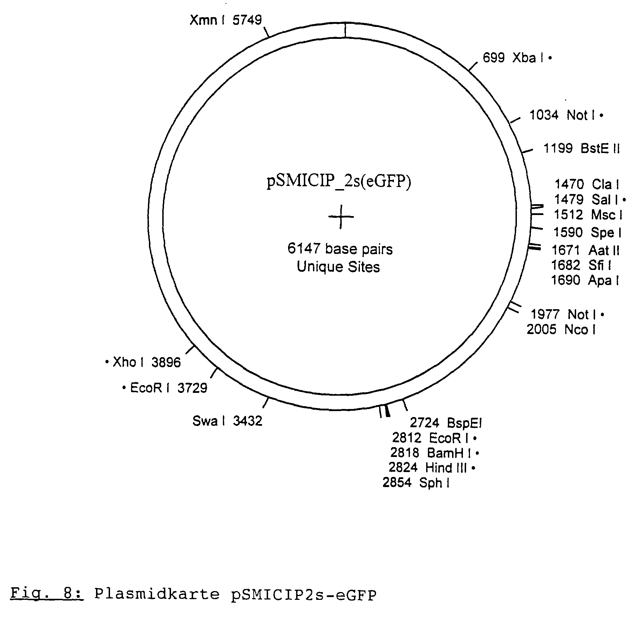

Es wurde das provirale Plasmid pSMICIP2s(eGFP), erhalten, dessen Plasmidkarte in Fig. 8 gezeigt ist. Eine Probe dieses Plasmids wurde am 31. August 2000 bei der Deutschen Sammlung von Mikroorganismen und Zellkulturen (DSMZ), Mascheroder Weg 1b, 38124 Braunschweig unter der Nr. DSM 13711 hinterlegt.

### Beispiel 2:

### Evaluierung der Verpackungseffizienz und Expressionseigenschaften:

Folgende Experimente wurden durchgeführt, um zu untersuchen, ob die erfindungsgemäße Insertion der Fremdsequenz 1 [FS1]
A) unter Erhalt der Verpackungseffizienz möglich ist und
B) eine Verbesserung der Expressionseigenschaften erlaubt.

### A) Erhalt der Verpackungseffizienz

Hintergrund: Replikationsdefekte retrovirale Vektoren werden in der Zellkultur in sicherheitsmodifizierten Verpackungszellen hergestellt. Eine gängige Verpackungszelle ist PHOENIX-ampho, die replikationsdefekte Mausretroviren mit amphotropem Hüllprotein freisetzt (Kinsella and Nolan, 1996). Bis zu 96 Stunden nach Transfektion der proviralen Vektorplasmide können Virusüberstände mit einem infektiösen Titer zwischen 10⁵ und 10⁶ Partikeln pro ml Zellkulturüberstand geerntet werden. Wie zuvor beschrieben (Fehse et al., 2000), wird ein zweites Plasmid kotransfiziert, das für das Hüllprotein des Vesicular-Stomatitis-Virus (VSV-G) kodiert. So können gemischte Virushüllen mit erweitertem Wirtsbereich erzeugt werden, wodurch die nachfolgende Transduktion verschiedener Zellarten erleichtert wird. Diese Modifikation hat keinen Einfluss auf die genetischen Eigenschaften des übertragenen Vektors (Laer et al., 1998). Entscheidend für den Erhalt der Verpackungseffizienz sind die Größe und die genetische Stabilität des Vektorgenoms sowie das Fehlen einer Interferenz mit der Funktion von Ψ. Nach dem Stand der Forschung war unbekannt, ob Fremdsequenzen in der hochkonservierten Region zwischen PBS und SD eingefügt werden können, ohne die für die Verpackung notwendige RNA-Sekundärstruktur zu beeinträchtigen (Alford et al., 1991).

Vorgehen: Die o.g. Plasmide wurden mit der Calciumphosphat-Transfektionsmethode zusammen mit einem VSV-G-Protein-kodierenden Plasmid in einen gleichmäßigen Rasen von PHOENIXampho-Zellen eingebracht. Innerhalb der nächsten 96 Stunden wurden fraktioniert Zellkulturüberstände abgenommen, filtriert (0.22 µm) und in Aliquots bei -70° konserviert. Der Gehalt an infektiösen Viruspartikeln wurde durch retrovirale Transduktion von mesenchymalen Mausfibroblasten der Linie SC1 bestimmt, indem eine konstante Zielzellzahl mit steigenden Mengen an virushaltigem Überstand versetzt wurde (Fehse et al., 2000). Die Fraktion der erfolgreich retroviral transduzierten Zellen wurde nach 48 Stunden über die Expression von EGFP im Durchflußzytometer bestimmt. Der maximale Virustiter wurde über den Mittelwert der besten fünf Ergebnisse ermittelt.

Ergebnis: Die Titer der Vektoren SMICIP2s(eGFP) (Titer 223800 ± 45400 / ml) und SINoMI-EGFP (Titer 237500 ± 55000 / ml) liegen in derselben Größenordnung wie der Titer des herkömmlichen Vektors SF110-EGFP (Titer 134700 ± 76700 / ml). Niedrigere Titer erzielen die Vektoren SINoM (43500 ± 13000 / ml) und SINoMS (31800 ± 64000 / ml).

Bewertung: Diese Experimente zeigen, dass der Einfluss der Fremdsequenz 1 [FS1] auf den Titer kontextabhängig ist, d.h. auch vom Aufbau der [FS2] und [FS3] abhängt. In Kombination mit der MAR in der Fremdsequenz 2 [FS2] lassen sich Standardtiter erzielen. Kontrollexperimente zeigen, dass die MAR-Sequenzen per se den Titer nicht heben. Daher ist die Erzielung ausreichender Virustiter mit dem erfindungsgemäßen Vektoraufbau auch in anderen Sequenzkombinationen möglich.

### B) Expressionseigenschaften

Hintergrund: Die Expression der übertragenen Gene ist ein wichtiges Kriterium für die Beurteilung der Qualität retroviraler Vektoren. Für die Expression vieler Transgene ist es förderlich, für einige sogar entscheidend, ein Intron in der 5'-untranslatierten Region zu platzieren (Buchmann und Berg, 1988; Hildinger et al., 1999). Das Intron muss allerdings bei der Expression der retroviralen RNA in der Verapckungszelle erhalten bleiben, da es sonst im übertragenen retroviralen Genom nicht mehr enthalten ist. Der Erhalt des Introns kann beispielsweise dadurch erreicht werden, dass Spleißdonor- und Spleißakzeptorsignale an den Flanken von Ψ eingefügt werden (Hildinger et al., 1999). Dies wurde bislang nur mit Vektoren praktiziert, die Enhancer-Promotorsequenzen in den LTRs tragen. Solche Vektoren haben den potentiellen Nachteil, dass es zur transkriptionellen Aktivierung stromabwärts gelegener zellulärer Sequenzen kommen kann, sofern der Enhancer-Promotor im 3'-LTR aktiviert wird. Daher ist es wünschenswert, das Prinzip des Intron-haltigen Transgens im Kontext eines Vektors umzusetzen, dessen Enhancer-Promotorbereich im LTR deletiert wurde (sog. selbstinaktivierender Vektor, Yu et al., 1986). Eine solche Konstruktion wurde erstmals in den erfindungsgemäßen Vektoren SMICIP2s(eGFP) und SINoM-EGFP realisiert.

Vorgehen: Zur Untersuchung der Expressionseigenschaften des SMCIP2s-EGFP Vektors wurden Mausfibroblasten der Linie SC1 und Mauslymphozyten der Linie EL4 unter Verwendung jeweils gleicher Mengen infektiöser Viruspartikel transduziert. Die Expressionshöhe von EGFP wurde nach 48 Stunden im Durchflußzytometer bestimmt. Pro Aufzeichnung wurden mind. 10000 unabhängige Ereignisse gemessen. Das mittlere Expressionsniveau wurde anhand des Mittelwertes (mittlere Fluroeszenz in willkürlichen Einheiten) aus mindestens zwei unabhängigen Experimenten ermittelt.

Ergebnis: In SC1-Zellen vermittelt der Vektor SMICIP2s(eGFP) von allen getesteten Vektoren die höchste Expression (mittlere Fluoresenzenz 682 ± 19). SINoM-EGFP, die Variante ohne MAR, liegt niedriger (475 ± 21). SINoMS-EGFP, die Variante ohne Intron und MAR, erreicht eine noch geringere Expression (183 ± 11) und ist diesbezüglich identisch mit dem herkömmlichen Vektor, SF110-EGFP (185 ± 2). Der Kontrollvektor SINoMI-EGFP entspricht dem klassischen Aufbau eines selbstinaktiverenden Vektors, da der Promotor ohne Intron direkt vor der cDNA liegt; dieser Vektor ist bezüglich der Expression dem Vektor SMICIP2s(eGFP) ebenfalls unterlegen (315 + 21).

Auch in EL4-Zellen vermitteln die erfindungsgemäßen Vektoren mit dem Enhancer-Promotor als Fremdsequenz 1 [FS1] und nachgeschalteten Intron die höchste Expression, allerdings zeigt sich hier kein fördernder Einfluss der MAR-Region; der Vektor SINoM-EGFP vermittelt die höchste Expression (713 ± 19), gefolgt von SMI-CIP2s(eGFP) (600 ± 14). SINoMS-EGFP, die Variante ohne Intron und MAR, erreicht eine deutlich geringere Expression (391 ± 2), ist aber noch besser als der herkömmliche Vektor, SF110-EGFP (185 ± 2). Auch die Kontrolle SINoMI-EGFP (391 ± 2) zeigt sich erneut als den Vektoren SMICIP2s(eGFP) und SINoM-EGFP unterlegen.

Bewertung: Diese Experimente zeigen, dass die erfindungsgemäße Insertion von Fremdsequenzen zwischen PBS und SD genutzt werden kann, um die Expressionseigenschaften retroviraler Vektoren zu verbessern. Die gewählte Konfiguration des Enhancer-Promotors unmittelbar stromaufwärts des retroviralen Introns kombiniert die sicherheitsrelevanten Vorteile eines selbstinaktivierenden Vektors (mit deletierten Enhancer-Promotorsequenzen des LTRs) mit den expressionsfördernden Eigenschaften eines Intron-haltigen Vektors. Der Expressionsgewinn um den Faktor 2 bis 3 gegenüber herkömmlichen Vektortypen zeigt sich in beiden Zellinien. Die Präsenz der MAR-Region hingegen war nur in den untersuchten Fibroblasten förderlich, was auf eine ausgeprägtere Differenzierungsabhängigkeit dieses Elementes hindeutet. Diese Befunde unterstreichen, dass der durch den neuartigen Vektoraufbau erzielte Expressionsgewinn nicht von der Präsenz einer MAR abhängt.

### C) Zusammenfassende Bewertung:

Der erfindungsgemäß gewählte Vektoraufbau erweitert die Möglichkeiten der Konstruktion retroviraler Gentransfervektoren. Es wurde experimentell gezeigt, dass
- eine Fremdgeninsertion zwischen der retroviralen PBS und dem Verpackungssignal möglich ist
- Vektoren mit diesem Aufbau in ausreichenden Titern produziert werden können
- in einer bevorzugten Ausführung die erfindungsgemäße Fremdsequenzinsertion genutzt werden kann, um Vektoren mit verbesserten Expressionseigenschaften und vorteilhaften, die biologische Sicherheit des Gentransfers potentiell fördernden Eigenschaften zu erzeugen.

### Referenzen:

ALFORD, R.L., HONDA, S., und BELMONT, J.W. (1991). RNA secondary structure of the packaging signal for Moloney murine leukemia virus. Virology **183**, 611-619.

BAUM, C., HEGEWISCH-BECKER, S., ECKERT, H.-G., STOCKING, C., und OSTERTAG, W. (1995). Novel retroviral vectors for efficient expression of the multidrug-resistance (mdr-1) gene in early hemopoietic cells. J. Virol. **69**, 7541-7547.

BUCHMAN, A.R. und BERG, P. (1988). Comparison of intron-dependent gene expreseeion. Mol. Cell. Biol. **8**, 4395-4405.

DEFFAUD, C. und DARLIX, J.L. (2000). Characterization of an internal ribosomal entry segment in the 5' leader of murine leukemia virus env RNA. J. Virol. **74**, 846-850.

FEHSE, B., RICHTERS, A., PUTIMTSEVA-SCHARF, K., KLUMP, H., LI, Z., OSTERTAG, W., ZANDER, A.R., und BAUM, C. (2000). CD34 splice-variant: an attractive marker for selection of gene-modified cells. Mol. Ther. **5**, 448-456.

HILDINGER, M., ABEL, K.L., OSTERTAG, W., und BAUM, C. (1999). Design of 5' untranslated sequences in retroviral vectors developed for medical use. J. Virol. **73**, 4083-4089.

KINSELLA, T.M., und NOLAN, G.P. (1996). Episomal vectors rapidly and stably produce high-titer recombinant retrovirus. Hum. Gene Ther. **7**, 1405-1413.

VON LAER, D., THOMSEN, S., VOGT, B., DONATH, M., KRUPPA, J., REIN, A., OSTERTAG, W., und STOCKING, C. (1998). Entry of amphotropic and 10A1 pseudotyped murine retroviruses is restricted in hematopoietic stem cell lines. J. Virol. **72**, 1424-1430.

SCHAMBACH, A., WODRICH, H., HILDINGER, M., BOHNE, J., KRÄUSSLICH, H.G., BAUM, C. (2000). Context-dependence of different modules for post-transcriptional enhancement of gene expression from retroviral vectors. Mol. Ther. **2**, 435-445.

SCHÜBELER, D., MIELKE, C., MAASS, K., und BODE, J. (1996). Scaffold/matrix-attachment regions act upon transcription in a context-dependent manner. Biochemistry 35, 11160-11169.

YANG, T.T., CHENG, L., und KAIN, S.R. (1996). Optimized codon usage and chromophore mutations provide enhanced sensitivity with the green fluorescent protein. Nucleic Acids Res. 24, 4592-4593.

YU, S.F., VON RUDEN, T., KANTOFF, P.W., GARBER, C., SEIBERG, M., RUTHER, U., ANDERSON, W.F., WAGNER, E.F., und GILBOA, E. (1986) Self-inactivating retroviral vectors designed for transfer of whole genes into mammalian cells. Proc Natl Acad Sci USA **83**:3194-3198.

ZUFFEREY, R., DONELLO, J.E., TRONO, D., und HOPE, T.J. (1999) Woodchuck hepatitis virus post-transcriptional regulatory element enhances expression of transgenes delivered by retroviral vectors. J. Virol. **73,** 2886-2892.

## Patentansprüche

1. Retroviraler Vektor mit dem allgemeinen Aufbau 5'-[LTR] - [PBS] - [SD] - [ψ] - [SA] - [PP] - [LTR]-3', bei dem
[LTR] die endständige Sequenzwiederholung *(long terminal repeat,* LTR) des retroviralen Vektors ist,
[PBS] die retrovirale Primerbindungsstelle ist,
[SD] das retrovirale Spleißdonorsignal ist,
[Ψ] das retrovirale Verpackungssignal ist,
[SA] das retrovirale Spleißakzeptorsignal ist,
und
[PP] ein Polypurintrakt (PP-Trakt) ist,
**dadurch gekennzeichnet, daß**
der Vektor zwischen der retroviralen Primerbindungsstelle (PBS) und dem retroviralen Spleißdonor (SD) eine weitere Fremdsequenz [FS1] enthält, die aus der Gruppe bestehend aus Enhancer-Promotor-Sequenzen, Enhancer-Sequenzen, cDNA-Sequenzen, Sequenzen für Chromatin-Modifikation, und/oder Sequenzen für RNA-Prozessierungssignale ausgewählt ist.

2. Vektor nach Anspruch 1, **dadurch gekennzeichnet, daß** er ferner eine zwischen [SA] und [PP] insertierte Fremdsequenz 2 [FS2] enthält, die aus der Gruppe bestehend aus cDNA-Sequenzen, Enhancer- und/oder Promotor-Sequenzen, Sequenzen für Interne Ribosomeneintrittstellen (IRES), Sequenzen für Chromatin-Modifikation und/oder Sequenzen für RNA-Prozessierungssignale ausgewählt ist.

3. Vektor nach Anspruch 1 oder 2, **dadurch gekennzeichnet, daß** die endständige Sequenzwiederholung (LTR) eine Fremdsequenz beinhaltet, die aus der Gruppe bestehend aus Klonierungsstellen zur Deletion des LTR-Enhancer-Promotors, Enhancer- und/oder Promotor-Sequenzen, cDNA-Sequenzen, Sequenzen für Chromatin-Modifikation und/oder Sequenzen für RNA-Prozessierungssignale ausgewählt ist.

4. Vektor nach den Ansprüchen 1 bis 3, **dadurch gekennzeichnet, daß** das retrovirale Spleißdonorsignal (SD) und/oder das retrovirale Spleißakzeptorsignal (SA) durch Mutation inaktiviert ist/sind.

5. Vektor nach den Ansprüchen 1 bis 4, **dadurch gekennzeichnet, daß** das Retrovirus aus der Gruppe bestehend aus Mausleukämieviren, Vogelretroviren, Spumaviren, B-Typ-Retroviren, D-Typ-Retroviren oder Lentiviren ausgewählt ist.

6. Vektor ensprechend DSM 13711.

7. Infektiöses Viruspartikel, **dadurch gekennzeichnet, daß** das Partikel den retroviralen Vektor nach den Ansprüchen 1 bis 6 enthält.

8. Wirtszelle, **dadurch gekennzeichnet, daß** sie mit dem retroviralen Vektor nach den Ansprüchen 1 bis 6 transfiziert ist.

9. Wirtszelle nach Anspruch 8, **dadurch gekennzeichnet, daß** sie mit einem infektiösen Viruspartikel nach Anspruch 7 infiziert ist.

10. Wirtszelle nach Anspruch 8, **dadurch gekennzeichnet, daß** sie mit einem Vektor entsprechend DSM 13711 transfiziert ist.

11. Wirtszelle nach den Ansprüchen 7 bis 10, **dadurch gekennzeichnet, daß** sie eine hämatopoetische, mesenchymale oder lymphatische Zelle ist.

12. Verwendung des retroviralen Vektors nach den Ansprüchen 1 bis 6 in der Gentherapie.

13. Verwendung des retroviralen Vektors nach den Ansprüchen 1 bis 6 zur Klonierung von Genen.

14. Verwendung des retroviralen Vektors nach den Ansprüchen 1 bis 6 zur Expression und/ oder Überexpression von Proteinen oder RNA.

15. Verwendung des retroviralen Vektors nach den Ansprüchen 1 bis 6 zur Transfektion hämatopoetischer, mesenchymaler oder lymphatischer Zellen.

16. Verfahren zur Gewinnung von Proteinen, **dadurch gekennzeichnet, daß** man eine Wirtszelle nach den Ansprüchen 8 bis 11 in einem geeigneten Medium unter Bedingungen kultiviert, die zur Expression der von den Fremdsequenzen [FS1], [FS2] und/oder [FS3] kodierten Proteine notwendig sind, wobei man das so erzeugte Protein von den Zellen und dem Medium abtrennt.

17. Verwendung des retroviralen Vektors nach den Ansprüchen 1 bis 6 zur Herstellung eines pharmazeutischen Präparates zur Gentherapie, **dadurch gekennzeichnet, daß** der Vektor in geeigneter Form vorliegt, um in die Zielzellen eingeführt zu werden.

18. Pharmazeutisches Präparat, **dadurch gekennzeichnet, daß** es einen Vektor nach den Ansprüchen 1 bis 6 zusammen mit pharmazeutisch verträglichen Hilfs- und Trägerstoffen enthält.


SEQUENZPROTOKOLL

<110> Heinrich-Pette-Institut

<120> Retrovirale Vektoren mit Fremdsequenzinsertion zwischen
      retroviraler Primerbindungsstelle und retroviralem
      Spleissdonor

<130> P057880

<140>
<141>

<160> 7

<170> PatentIn Ver. 2.1

<210> 1
<211> 6147
<212> DNA
<213> Kuenstliche Sequenz

<220>
<223> Beschreibung der kuenstlichen Sequenz: Plasmid
      pSMICIP2s(eGFP)

<400> 1
ctgcctcgcg cgtttcggtg atgacggtga aaacctctga cacatgcagc tcccggagac 60
ggtcacagct tgtctgtaag cggatgccgg gagcagacaa gcccgtcagg gcgcgtcagc 120
gggtgttggc gggtgtcggg gcgcagccat gacccagtca cgtagcgata gttactatgc 180
ggcatcagag cagattgtac tgagagtgca ccatatgcgg tgtgaaatac cgcacagatg 240
cgtaaggaga aaataccgca tcaggcgcca ttcgccattc aggctgcgca actgttggga 300
agggcgatcg gtgcgggcct cttcgctatt acgccagctg gcgaaagggg gatgtgctgc 360
aaggcgatta agttgggtaa cgccagggtt ttcccagtca cgacgttgta aaacgacggc 420
cagtgaatta gtactctagc ttaagtaagc cattttgcaa ggcatggaaa aatacataac 480
tgagaataga gaagttcaga tcaaggttag gaacagagag acaggagaat atgggccaaa 540
caggatatct gtggtaagca gttcctgccc cggctcaggg ccaagaacag ttggaacagc 600
agaatatggg ccaaacagga tatctgtggt aagcagttcc tgccccggct cagggccaag 660
aacagatggt ccccagatgc ggtcccgccc tcagcagttc tagagaacca tcagatgttt 720

ccagggtgcc ccaaggacct gaaatgaccc tgtgccttat ttgaactaac caatcagttc 780
gcttctcgct tctgttcgcg cgcttctgct ccccgagctc aataaaagag cccacaaccc 840
ctcactcggc gcgccagtcc tccgattgac tgagtcgccc gggtacccgt gttctcaata 900
aaccctcttg cagttgcatc cgactcgtgg tctcgctgat ccttgggagg gtctcctcag 960
attgattgac tgcccacctc gggggtcttt catttggagg ttccaccgag atttggagac 1020
ccctgcccgt cgagcggccg ctagctgcag taacgccatt ttgcaaggca tggaaaaata 1080
ccaaaccaag aatagagaag ttcagatcaa gggcgggtac atgaaaatag ctaacgttgg 1140
gccaaacagg atatctgcgg tgagcagttt cggccccggc ccggggccaa gaacagatgg 1200
tcaccgcagt ttcggccccg gcccgaggcc aagaacagat ggtccccaga tatggcacaa 1260
ccctcagcag tttcttaaga cccatcagat gtttccaggc tcccccaagg acctgaaatg 1320
accctgcgcc ttatttgaat taaccaatca gcctgcttct cgcttctgtt cgcgcgcttc 1380
tgcttcccga gctctataaa agagctcaca acccctcact cggcgcgcca gtcctccgac 1440
agactgagtc ggccggtcga atcaagctca tcgataccgt cgaccaccga cccccccgcc 1500
gggaactaag ctggccagcg gtcgtttcgt gtctgtctct gtctttgtgc gtgtttgtgc 1560
cggcatctaa tgtttgcgcc tgcgtctgta ctagttggct aactagatct gtatctggcg 1620
gtcccgcgga agaactgacg agttcgtatt cccggccgca gcccctggga gacgtcccag 1680
cggcctcggg ggcccgtttt gtggcccatt ctgtatcagt taacctaccc gagtcggact 1740
ttttggagct ccgccactgt ccgaggggta cgtggctttg ttgggggacg agagacagag 1800
acacttcccg cccccgtctg aatttttgct ttcggtttta cgccgaaacc gcgccgcgcg 1860
tcttgtctgc tgcagcatcg ttctgtgttg tctctgtctg actgtgtttc tgtatttgtc 1920
tgaaaattag ctcgacaaag ttaagtaata gtccctctct ccaagctcac ttacaggcgg 1980
ccgctctagc gctaccggtc gccaccatgg tgagcaaggg cgaggagctg ttcaccgggg 2040
tggtgcccat cctggtcgag ctggacggcg acgtaaacgg ccacaagttc agcgtgtccg 2100
gcgagggcga gggcgatgcc acctacggca agctgaccct gaagttcatc tgcaccaccg 2160
gcaagctgcc cgtgccctgg cccaccctcg tgaccaccct gacctacggc gtgcagtgct 2220
tcagccgcta ccccgaccac atgaagcagc acgacttctt caagtccgcc atgcccgaag 2280
gctacgtcca ggagcgcacc atcttcttca aggacgacgg caactacaag acccgcgccg 2340
aggtgaagtt cgagggcgac accctggtga accgcatcga gctgaagggc atcgacttca 2400
aggaggacgg caacatcctg gggcacaagc tggagtacaa ctacaacagc cacaacgtct 2460
atatcatggc cgacaagcag aagaacggca tcaaggtgaa cttcaagatc cgccacaaca 2520
tcgaggacgg cagcgtgcag ctcgccgacc actaccagca gaacaccccc atcggcgacg 2580
gccccgtgct gctgcccgac aaccactacc tgagcaccca gtccgccctg agcaaagacc 2640
ccaacgagaa gcgcgatcac atggtcctgc tggagttcgt gaccgccgcc gggatcactc 2700
tcggcatgga cgagctgtac aagtccggac tcagatccag cgctgataac ccagcctcca 2760
ccacaaacaa ggatgccggt accggcgccc accaccacca ccaccacatg tgaattcgga 2820
tccaagcttt ttctctacac tgaagtcatg atggcatgct tctatattat tttctaaaag 2880
atttaaagtt ttgccttctc catttagact tataattcac tggaattttt ttgtgtgtat 2940
ggtatgacat atgggttccc ttttattttt tacatataaa tatatttccc tgtttttcta 3000
aaaaagaaaa agatcatcat tttcccattg taaaatgcca tatttttttc ataggtcact 3060
tacatatatc aatgggtctg tttctgagct ctactctatt ttatcagcct cactgtctat 3120

ccccacacat ctcatgcttt gctctaaatc ttgatattta gtggaacatt ctttcccatt 3180
ttgttctaca agaatatttt ttgttaattg tcttttgggc ttcctatata cattttagaa 3240
tgaggttggc aagttaacaa acagcttttt tggggtgaac atattgacta caaatttatg 3300
tggaaagaaa gtataccttc acaatattaa gtcttttagt tcatgaatat agtatgtctc 3360
tccgtttctg cattaactta gacattcatt aatttctctc acaatttata agtttattta 3420
gatcttcatt catttaaatc ttcactaacc tctcatttac aatttgtaag ttttctgggt 3480
aacagtcttg cacttctttg cctagattta tttccaagta gattattttc atacatcgtc 3540
tatggtgtca tttttaaaat gtaatttttc acctttttat tgctaaagag agatgactga 3600
ttgttaatat tgatcttgtg cgtggcgacc ttgctgaatt agcttaacac gagccataga 3660
tagaataaaa gattttattt agtctccaga aaaagggggg aatgaaagac cccaccgcta 3720
gcgatatcga attcacaacc cctcactcgg cgcgccagtc ctccgacaga ctgagtcgcc 3780
cgggtacccg tgttctcaat aaaccctctt gcagttgcat ccgactcgtg gtctcgctgt 3840
tccttgggag ggtctcctct gagtgattga ctgcccacct cgggggtctt tcattctcga 3900
gagctttggc gtaatcatgg tcatagctgt ttcctgtgtg aaattgttat ccgctcacaa 3960
ttccacacaa catacgagcc ggaagcataa agtgtaaagc ctggggtgcc taatgagtga 4020
gctaactcac attaattgcg ttgcgctcac tgcccgcttt ccagtcggga aacctgtcgt 4080
gccagctgca ttaatgaatc ggccaacgcg cggggagagg cggtttgcgt attgggcgct 4140
cttccgcttc ctcgctcact gactcgctgc gctcggtcgt tcggctgcgg cgagcggtat 4200
cagctcactc aaaggcggta atacggttat ccacagaatc aggggataac gcaggaaaga 4260
acatgtgagc aaaaggccag caaaaggcca ggaaccgtaa aaaggccgcg ttgctggcgt 4320
ttttccatag gctccgcccc cctgacgagc atcacaaaaa tcgacgctca agtcagaggt 4380
ggcgaaaccc gacaggacta taaagatacc aggcgtttcc ccctggaagc tccctcgtgc 4440
gctctcctgt tccgaccctg ccgcttaccg gatacctgtc cgcctttctc ccttcgggaa 4500
gcgtggcgct ttctcaatgc tcacgctgta ggtatctcag ttcggtgtag gtcgttcgct 4560
ccaagctggg ctgtgtgcac gaaccccccg ttcagcccga ccgctgcgcc ttatccggta 4620
actatcgtct tgagtccaac ccggtaagac acgacttatc gccactggca gcagccactg 4680
gtaacaggat tagcagagcg aggtatgtag gcggtgctac agagttcttg aagtggtggc 4740
ctaactacgg ctacactaga aggacagtat ttggtatctg cgctctgctg aagccagtta 4800
ccttcggaaa aagagttggt agctcttgat ccggcaaaca aaccaccgct ggtagcggtg 4860
gtttttttgt ttgcaagcag cagattacgc gcagaaaaaa aggatctcaa gaagatcctt 4920
tgatcttttc tacggggtct gacgctcagt ggaacgaaaa ctcacgttaa gggattttgg 4980
tcatgagatt atcaaaaagg atcttcacct agatcctttt aaattaaaaa tgaagtttta 5040
aatcaatcta aagtatatat gagtaaactt ggtctgacag ttaccaatgc ttaatcagtg 5100
aggcacctat ctcagcgatc tgtctatttc gttcatccat agttgcctga ctccccgtcg 5160
tgtagataac tacgatacgg gagggcttac catctggccc cagtgctgca atgataccgc 5220
gagacccacg ctcaccggct ccagatttat cagcaataaa ccagccagcc ggaagggccg 5280
agcgcagaag tggtcctgca actttatccg cctccatcca gtctattaat tgttgccggg 5340
aagctagagt aagtagttcg ccagttaata gtttgcgcaa cgttgttgcc attgctgctg 5400
gcatcgtggt gtcacgctcg tcgtttggta tggcttcatt cagctccggt tcccaacgat 5460
caaggcgagt tacatgatcc cccatgttgt gcaaaaaagc ggttagctcc ttcggtcctc 5520

cgatcgttgt cagaagtaag ttggccgcag tgttatcact catggttatg gcagcactgc 5580
ataattctct tactgtcatg ccatccgtaa gatgcttttc tgtgactggt gagtactcaa 5640
ccaagtcatt ctgagaatag tgtatgcggc gaccgagttg ctcttgcccg gcgtcaatac 5700
gggataatac cgcgccacat agcagaactt taaaagtgct catcattgga aaacgttctt 5760
cggggcgaaa aaccagccag ccggaagggc cgagcgcaga agtggtcctg caactttatc 5820
cgcctccatc cagtctatta attgttgccg ggaagctaga gtaagtagtt cgccagttaa 5880
tagtttgcgc aacgttgttg ccattgctgc tggcatcgtg gtgtcacgct cgtcgtttgg 5940
tatggcttca ttcagctccg gttcccaacg atcaaggcga gttacatgat cccccatgtt 6000
gtgcaaaaaa gcggttagct ccttcggtcc tccgatcgtt gtcagaagta agttggccgc 6060
agtgttatca ctcatggtta tggcagcact gcataattct cttactgtca tgccatccgt 6120
aagatgcttt tctgtgactg gtgagta 6147

<210> 2
<211> 17
<212> DNA
<213> Kuenstliche Sequenz

<220>
<223> Beschreibung der kuenstlichen Sequenz: Primer M13
forward

<400> 2
tgaccggcag caaaatg 17

<210> 3
<211> 19
<212> DNA
<213> Kuenstliche Sequenz

<220>
<223> Beschreibung der kuenstlichen Sequenz: Primer M13
reverse

<400> 3
ggaaacagct atgaccatg 19

<210> 4
<211> 26
<212> DNA
<213> Kuenstliche Sequenz

<220>
<223> Beschreibung der kuenstlichen Sequenz: Primer
      SalEagSF63

<400> 4
gtcgaccggc cgactcagtc aatcgg                                          26


<210> 5
<211> 32
<212> DNA
<213> Kuenstliche Sequenz

<220>
<223> Beschreibung der kuenstlichen Sequenz: Primer U3EcoRI

<400> 5
atcgaattca caacccctca ctcggcgcgc ca                                   32


<210> 6
<211> 32
<212> DNA
<213> Kuenstliche Sequenz

<220>
<223> Beschreibung der kuenstlichen Sequenz: Primer U3NheI

<400> 6
atcgctagcg gtggggtctt tcattccccc ct                                   32

<210> 7
<211> 28
<212> DNA
<213> Kuenstliche Sequenz

<220>
<223> Beschreibung der kuenstlichen Sequenz: Primer
      XhoEagSF65

<400> 7
ctcgagcggc cgctagctgc agtaacgc                                    28